# EUROPEAN PATENT SPECIFICATION

(11) **EP 4 003 393 B1**
(45) Date of publication and mention of the grant of the patent: **04.11.2026**
(21) Application number: 20754570.8
(22) Date of filing: 28.07.2020
(51) Int. Cl.: A61K 38/10, A61P 17/02, C07K 7/08

(54) **COMPOSITION AND METHOD FOR PROMOTING WOUND HEALING**
ZUSAMMENSETZUNG UND VERFAHREN ZUR FÖRDERUNG DER WUNDHEILUNG
COMPOSITION ET MÉTHODE POUR FAVORISER LA GUÉRISON DES BLESSURES

(30) Priority: 29.07.2019 US 201962879717 P
(43) Date of publication of application: 01.06.2022
(73) Proprietor: THE BOARD OF TRUSTEES OF THE UNIVERSITY OF ILLINOIS, Urbana, IL 61801 (US)
(72) Inventor: MINSHALL, Richard, D., Indian Head Park, IL 60525 (US); CHEN, Zhenlong, Chicago, IL 60607 (US)
(74) Representative: FRKelly
(86) International application number: PCT/US2020/043813
(87) International publication number: WO 2021/021774

(56) References cited:
- WO-A1-00/28076
- WO-A1-2009/012435
- MICHEL JEFFREY B ET AL: "Caveolin versus calmodulin. Counterbalancing allosteric modulators of endothelial nitric oxide synthase", JOURNAL OF BIOLOGICAL CHEMISTRY, AMERICAN SOCIETY FOR BIOCHEMISTRY AND MOLECULAR BIOLOGY, US, vol. 272, no. 41, 10 October 1997 (1997-10-10), pages 25907 - 25912, XP002136661, ISSN: 0021-9258, DOI: 10.1074/JBC.272.41.25907
- WU PEI-RUNG ET AL: "Lobe-Specific Calcium Binding in Calmodulin Regulates Endothelial Nitric Oxide Synthase Activation", PLOS ONE, vol. 7, no. 6, 29 June 2012 (2012-06-29), US, pages e39851, XP093203588, ISSN: 1932-6203, DOI: 10.1371/journal.pone.0039851
- ALLISON R. NELSON ET AL: "Myristoyl-Based Transport of Peptides into Living Cells +", BIOCHEMISTRY, vol. 46, no. 51, 1 December 2007 (2007-12-01), pages 14771 - 14781, XP055398482, ISSN: 0006-2960, DOI: 10.1021/bi701295k
- MICHAEL PIAZZA ET AL: "Solution Structure of Calmodulin Bound to the Target Peptide of Endothelial Nitric Oxide Synthase Phosphorylated at Thr495", BIOCHEMISTRY, vol. 53, no. 8, 17 February 2014 (2014-02-17), pages 1241 - 1249, XP055740964, ISSN: 0006-2960, DOI: 10.1021/bi401466s
- MICHAEL PIAZZA ET AL: "Structural Studies of a Complex Between Endothelial Nitric Oxide Synthase and Calmodulin at Physiological Calcium Concentration", BIOCHEMISTRY, vol. 55, no. 42, 12 October 2016 (2016-10-12), pages 5962 - 5971, XP055740967, ISSN: 0006-2960, DOI: 10.1021/acs.biochem.6b00821
- ZHENLONG CHEN ET AL: "CCL28-induced CCR10/eNOS interaction in angiogenesis and skin wound healing", THE FASEB JOURNAL, vol. 34, no. 4, 2 March 2020 (2020-03-02), US, pages 5838 - 5850, XP055740894, ISSN: 0892-6638, DOI: 10.1096/fj.201902060R
- ERICH BÜNEMANN ET AL: "Chemokine ligand-receptor interactions critically regulate cutaneous wound healing", EUROPEAN JOURNAL OF MEDICAL RESEARCH, BIOMED CENTRAL LTD, LONDON, UK, vol. 23, no. 1, 16 January 2018 (2018-01-16), pages 1 - 17, XP021252827, DOI: 10.1186/S40001-017-0299-0

## Description

### Background

Cardiovascular complications of type 2 diabetes mellitus (T2DM), which is now the third leading cause of death in the United States, greatly reduce the quality of life and likely forms the basis for the 25% lifetime risk of developing non-healing diabetic foot ulcers (DFUs) which precede amputation in up to 90% of diabetic amputees. DFUs are associated with significant health care costs estimated to be upwards of 10 billion dollars/year in the United Sates alone.

Current interventions for treating DFUs include the use of bioengineered skin equivalents, growth factors, granulocyte colony-stimulation factor, hyperbaric oxygen therapy, and negative pressure wound therapy. However, in a meta-analysis of randomized-controlled trials, evidence does not support the above interventions such as growth factors as being beneficial for DFU patients (Martí-Carvajal, et al. (2015) Cochrane Database Syst. Rev. 10:CD008548) and thus there remains an enormous unmet need.

MICHAEL PIAZZA ET AL: "Solution Structure of Calmodulin Bound to the Target Peptide of Endothelial Nitric Oxide Synthase Phosphorylated at Thr495", BIOCHEMISTRY, vol. 53, no. 8, 17 February 2014, pages 1241-1249, ISSN: 0006-2960, DOI: 10.1021/bi401466s discloses the human eNOS of amino acid sequence TRKKTFKEVANAVKISASLMGT and the Thr495phosphorylated human eNOS TRKKpTFKEVANAVKISASLM.

MICHAEL PIAZZA ET AL: "Structural Studies of a Complex Between Endothelial Nitric Oxide Synthase and Calmodulin at Physiological Calcium Concentration", BIOCHEMISTRY, vol. 55, no. 42, 12 October 2016 (2016-10-12), pages 5962-5971, ISSN: 0006-2960, DOI: 10.1021/acs.biochem.6b00821 discloses the human eNOS amino acid sequence TRKKTFKEVANAVKISASLMGT.

WO 00/28076 A1 discloses the peptide sequence derived from eNOS which comprises the amino acid sequence TRKKTpFKEVANAVKISASLM and located around the Thr-495, in the CaM-binding sequence between the oxidase and reductase domains of eNOS.

WO 2009/012435 A1 discloses six calmodulin-binding peptides including the amino acid sequence of RKKTFKEVANAVKISASLMG.

ERICH BUNEMANN ET AL: "Chemokine ligand-receptor interactions critically regulate cutaneous wound healing", EUROPEAN JOURNAL OF MEDICAL RESEARCH, BIOMED CENTRAL LTD, LONDON, UK, vol. 23, no. 1, 16 January 2018 (2018-01-16), pages 1-17, DOI: 10.1186/S40001-017-0299-0 discloses that CCL27 markedly enhances wound repair of structural skin cells.

MICHEL JEFFREY B ET AL: "Caveolin versus calmodulin. Counterbalancing allosteric modulators of endothelial nitric oxide synthase", JOURNAL OF BIOLOGICAL CHEMISTRY, AMERICAN SOCIETY FOR BIOCHEMISTRY AND MOLECULAR BIOLOGY, US, vol. 272, no. 41, 10 October 1997 (1997-10-10), pages 25907-25912, ISSN: 0021-9258, DOI: 10.1074/JBC.272.41.25907 discloses the calmodulin-binding domain of bovine eNOS, TRKKTFKEVANAVKISASLM.

WU PEI-RUNG ET AL: "Lobe-Specific Calcium Binding in Calmodulin Regulates Endothelial Nitric Oxide Synthase Activation", PLOS ONE, vol. 7, no. 6, 29 June 2012 (2012-06-29), page e39851, ISSN: 1932-6203, DOI: 10.1371/journal.pone.0039851 discloses a peptide described as a canonical CaM-binding peptide (CBD), TRKKTFKEVANAVKISASLM.

ALLISON R. NELSON ET AL: "Myristoyl-Based Transport of Peptides into Living Cells+", BIOCHEMISTRY, vol. 46, no. 51, 1 December 2007 (2007-12-01 ), pages 14771-14781, ISSN: 0006-2960, DOI: 10.1021/bi701295k discloses the myristoylation of peptides in order to transport them into living cells.

### Summary of the Invention

This invention provides a construct consisting of myr-KISASLM (SEQ ID NO:64). This invention further provides a pharmaceutical composition including the construct as well as a method of promoting or accelerating wound healing in a subject, e.g., a subject with diabetes.

### Brief Description of the Drawings

FIG. 1 shows a time course of wound healing in *WT* C57BL/6 mice treated with Myr-CBD7 construct. Four 5 mm full thickness excisional wounds were made on the mouse dorsal skin. Wounds were treated with 30 µl (50 µM) Myr-CBD7 (CBD7) construct or Myr-control construct (Ctl-P) per wound. Note that Myr-CBD7 construct treatment reduced wound size significantly on day 5 and 7. * *P* < 0.05 (n=8).
FIG. 2 shows re-epithelialization of skin wounds of *WT* mice with Myr-CBD7 treatment compared with control construct at day 7. The wound closure was measured and quantified in the respective conditions. * *P* < 0.05 (n=5).
FIG. 3 shows enhanced eNOS expression by Myr-CBD7 construct compared with control construct in mouse wound at day 7. * *P* < 0.05; ** *P* < 0.005 (n=3-4).
FIG. 4 shows that in co-IP experiments, CCR10-eNOS interactions are reduced in mouse wounds after 7 days of treatment with 50 µM Myr-CBD7 construct.
FIG. 5 shows Myr-CBD7 improves wound healing in db/db mice. Four 5 mm full thickness excisional wounds were made on *db*/*db* mouse dorsal skin. Immediately after punching, 30 µl (50 µM) Myr-control construct (Ctl-P) or Myr-CBD7 construct (CBD7) were topically applied to the wounds. Normalized wound size (day 0 was set as 100%) values are shown. * *P* < 0.05; ** *P* < 0.005; *** *P* < 0.001 vs Ctl-P (n=10-12). Note that Myr-CBD7 construct treatment reduces wound size significantly from day 4, compared with control construct.
FIG. 6 shows that disruption of eNOS-CCR10 interaction, results in elevated eNOS expression in *db*/*db* mouse wounds on day 12 after topical application of Myr-CBD7. Normalized values between eNOS and CCR10 are shown. ** *P* < 0.005 vs Ctl-P (n=5). Note that eNOS-CCR10 interaction is disrupted by Myr-CBD7, compared with Ctl-P, in *db*/*db* mouse wounds on day 12.

### Detailed Description of the Invention

Cardiovascular complications of type 2 diabetes mellitus (T2DM), greatly reduce the quality of life and likely forms the basis for the 25% lifetime risk of developing non-healing diabetic foot ulcers (DFUs) which precede amputation in up to 90% of cases. In patients with DFUs, persistent hyperglycemia leads to endothelial cell (EC) dysfunction which results in defective endothelial nitric oxide synthase (eNOS) activity and decreased nitric oxide (NO) production. eNOS is constitutively expressed in vascular endothelial cells and plays an important role in the regulation of vascular tone and angiogenesis. It has been reported that deficits in the wound healing response in obese mice can be overcome by increasing NO production via overexpression of eNOS.

It has now been found that punch biopsies from human subjects with T2DM and obesity-induced diabetes in mice also exhibit an increase in expression of chemokine receptor CCR10, eNOS sequestration and inactivation by CCR10 and reduction in eNOS expression and NO bioavailability. Accordingly, a cell-permeable construct has now been developed that targets CCR10-eNOS binding thereby increasing eNOS/NO levels and improving skin wound healing in obese and diabetic mice. The novel construct, designated Myr-CBD7 (myristoylated CCR10 binding domain), can prevent CCR10-eNOS interaction and also upregulate eNOS expression and activity in dysfunctional endothelial cells. Myr-CBD7 can be provided at concentrations up to 100 µM without adversely affecting cell viability. The construct efficiently enters vascular endothelial cells resulting in rescue of normal endothelial cell function and improves skin wound healing in obese and diabetic mice. Therefore, this construct finds use as a novel therapy for improving skin wound healing, in particular in diabetic patients.

Accordingly, the present invention provides a construct consisting of myr-KISASLM (SEQ ID NO:64), and use of the same in compositions and methods for modulating the CCR10-eNOS interaction and promoting wound healing. Ideally, the construct of the invention does not interfere with or disrupt binding of calmodulin and eNOS. For the purposes of this invention, the term "construct" is used herein to refer to the peptide consisting of amino acid sequence KISASLM (SEQ ID NO: 64) that targets CCR10-eNOS binding, which has been modified by recombinant, chemical and/or enzymatic techniques to include one N-terminal myristoyl group (myr) carrier moiety that enhances uptake of the peptide. In particular, at least one of the one or more carrier moieties is not naturally associated with the peptide. The construct of the invention is the construct consisting of myr-KISASLM (SEQ ID NO: 64).

The term "peptide," as used herein in this disclosure, refers broadly to a sequence of two or more amino acids joined together by peptide bonds. It should be understood that this term does not connote a specific length of a polymer of amino acids, nor is it intended to imply or distinguish whether the peptide is produced using recombinant techniques, chemical or enzymatic synthesis or is naturally occurring. Ideally, the CBD peptide provides a minimum binding site to disrupt the CCR10-eNOS interaction. In particular, the peptide comprises or consists of the amino acid sequence Lys-Ile-Ser-Ala-Ser-Leu-Met (SEQ ID NO: 1). Comparative peptides of the disclosure include the sequences KISASLM (SEQ ID NO:1), TRKKTFKEVANAVKISASLM (SEQ ID NO:2), FKEVANAVKISASLM (SEQ ID NO:3), and VANAVKISASLM (SEQ ID NO:4).

As used herein in this disclosure, "carrier moiety" refers to a polypeptide, polynucleotide, carbohydrate, or organic or inorganic molecule that facilitates traversing a lipid bilayer, micelle, cell membrane, organelle membrane, or vesicle membrane. A carrier moiety attached to another molecule facilitates the molecule traversing a membrane, for example going from extracellular space to intracellular space, or cytosol to within an organelle. In the present invention, a myr carrier moiety is covalently linked tc the amino terminus of the CBD peptide to form the construct myr-KISASLM (SEQ ID NO: 64).

The CBD peptide includes the lipid myristic acid to facilitate cell penetration. As used herein, lipids generally refer to water insoluble molecules soluble in organic solvents. A lipid may be a fatty acid, which includes an aliphatic hydrocarbon chain with an acyl group, where the aliphatic chain is either a saturated or an unsaturated alkyl with one or more double bonds. Typical fatty acids include, without limitation, lauric acid, myristic acid, palmitic acid, stearic acid, oleic acid, linoleic acid, valeroic acid, octanoic acid, decanoic acid and linolenic acid. Fatty acids are or could be linked to acyl group carriers, such as glycerol, sphingosine, cholesterol, and others. The lipophilic group may be attached to the peptide either directly or via a linking group. For example, (not claimed) 5-amino valeroic acid, 8-amino octanoic acid or 2-amino decanoic acid may be attached to the N- and/or C-terminus of the peptide.

Lipids can also be classified into different lipid classes based on their polarity. Lipids may be nonpolar or polar-lipids. Examples of such non-polar lipids are mono-, di- or triacylglycerols (glycerides), alkyl esters of fatty acids, and fatty alcohols. Polar lipids have polar head groups and exhibit surface activity, such as fatty amines, phosphatidic acid (*e.g*., phosphatidyl ethanolamine, phosphatidyl choline, etc.), phospholipids, glycolipids glycosylphosphatidylinositol), and the like. In certain forms, the lipids are attached or linked to nucleosides, nucleotides, nucleic acids, amino acid, proteins, or saccharides. Preferably, a CBD peptide is myristylated at the N terminal amino acid residue. This modification can be added either co-translationally or post-translationally with, e.g., N-myristoyltransferase (NMT) which catalyzes the myristic acid addition. In certain embodiments, the peptide is myristoylated at the N-terminal amino acid residue in order to facilitate entry of the peptide into the cell.

In certain aspects, the construct of the invention and comparative peptides are presented in Table 2. Only **myr-KISASLM** (SEQ ID NO:64) is according to the claimed invention; the remaining constructs are comparative/reference examples.

**TABLE 2**

| Construct | SEQ ID NO: |
|---|---|
| myr-TRKKTFKEVANAVKISASLM | 61 |
| myr-FKEVANAVKISASLM | 62 |
| myr-VANAVKISASLM | 63 |
| myr-KISASLM | 64 |
| RQIKIWFQNRRMKWKKKISASLM | 65 |
| KISASLMGLRKRLRKFRNKIKEK | 66 |
| KISASLMGLRKRLRKFRNKIKEK-NH₂ | 67 |
| KISASLMCRKDK | 68 |
| KISASLMCRKDK-NH₂ | 69 |

Comparative CBP peptides and constructs not forming part of the invention can be synthesized recombinantly using recombinant DNA techniques. Thus, herein disclosed are polynucleotides that encode such peptides or constructs. Also described are vectors, particularly expression vectors that harbor the polynucleotides encoding the comparative CBD peptide or construct. The vector provides replication, transcription and/or translation regulatory sequences that facilitate recombinant synthesis of the comparative CBD peptide or construct in eukaryotic or prokaryotic cells. Herein described are also host cells for recombinant expression of the comparative CBD peptide or construct and methods of harvesting and purifying the comparative CBD peptide or construct produced by the host cells. Production and purification of recombinant polypeptides is routine practice to one of skilled in the art. The CBD peptide or construct can be purified by any suitable methods known in the art including without limitation gel filtration and affinity purification. When the CBD peptide or construct is produced in the form of a fusion protein, the fusion moiety (or the epitope tag) can optionally be cleaved off using a protease before further analysis.

Alternatively, the construct of the invention can be advantageously synthesized by any of the chemical synthesis techniques known in the art, particularly solid-phase synthesis techniques, for example, using commercially-available automated peptide synthesizers. See, for example, Stewart and Young, 1984, Solid Phase Peptide Synthesis, 2nd ed., Pierce Chemical Co.; Tarn, et al. (1983) J. Am. Chem. Soc. 105:6442; Merrifield (1986) Science 232:341-347; and US 5,424,398). Moreover, a combination of recombinant and chemical synthesis techniques is also contemplated.

The construct of the invention can be purified by any suitable methods known in the art including, *e.g.,* affinity chromatography, ion exchange chromatography, filter, ultrafiltration, gel filtration, electrophoresis, salting out, dialysis, and the like. In one embodiment, the construct is purified by reverse-phase chromatography.

As demonstrated herein, the construct of this invention disrupts the eNOS-CCR10 interaction, increases eNOS/NO levels, increases re-epithelialization of skin wounds and improves skin wound healing in obese and diabetic mice. Accordingly, this invention provides the construct/composition according to the claims, for use in a method of promoting or accelerating wound healing, by modulating the CCR10-eNOS interaction and promoting, accelerating or improving wound healing in a subject. This involves administering to a subject in need of treatment (e.g. a subject with a wound such as a chronic wound or an infected wound) an effective amount of the construct. There are a variety of ways to measure wound healing. Often images are taken to calculate linear dimensions, perimeter and area. The NIH has a free program, Image J, that allows measurement of wound areas from an image. The final healing prognosis can be extrapolated from initial healing rates based on the migration of the periphery towards the center. This is done using a number of mathematical equations, the most common of which is a modified Gilman's equation. In addition to visual inspection, wound healing measurement can also be aided by spectroscopic methods or MRI. See, e.g., Dargaville, et al.

(2013) Biosensors Bioelect. 41:30-42; Tan, et al. (2007) Br. J. Radiol. 80:939-48. If healing is slow/inadequate, biopsies of the wound edges may be taken to rule out or determine infection and malignancy. In certain embodiments, the acceleration or improvement of wound healing can be assessed by comparing wound closure in treated and control wounds. In certain embodiments, the acceleration or improvement of wound healing is at least 20%, 30%, 40%, 50%, 60%, 70%, 80% or 90% faster or better than the control.

In certain aspects, the invention provides the construct/composition according to the claims, for use in methods for promoting/accelerating/improving healing of a wound with or without active infection, microbial contamination or colonization in the wound. The instant construct can be used for treating infected wounds or promoting/accelerating/improving infected wound healing. In certain embodiments, the construct can be used for treating wounds, or promoting/accelerating/improving wound healing, in the presence of infection. In some embodiments, the construct can be used for treating wounds or promoting/accelerating/improving wound healing in the presence of microbial contamination or colonization with risk for infection. In further embodiments, the patient in need of wound healing treatment can be a diabetic patient. Accordingly, in some embodiments, the wound is a diabetic wound, for example, diabetic foot ulcer. In some further embodiments, the wound is an infected diabetic wound, for example, infected diabetic foot ulcer.

For accelerating chronic wound healing, such as for the treatment of diabetic foot ulcer, the administration of the construct can be combined with one or more additional wound healing agents. Suitable additional wound healing agents include without limitation growth factors (*e.g*., EGF, FGF, IGF, PDGF, TGF, and VEGF), nerve growth factor (NGF), angiogenesis factors (*e.g*., HGF, TNF-α, angiogenin, IL-8, angiopoietins 1 and 2, Tie-2, integrin α5, matrix metalloproteinases, nitric oxide, COX-2), members of the platelet derived growth factor (PDGF) family (*e.g*., PDGF-A, PDGF-B, PDGF-C, and PDGF-D), members of the insulin growth factor (IGF) family (*e.g*., IGF-I, IGF-II), members of the transforming growth factor (TGF) family (e.g., TGF-α TGF-β) and anabolic oxygen (vacuum therapy). In certain embodiments, the construct can be co-administered with one or more additional wound healing agents described herein and/or one or more antibacterial agents or antibiotics suitable for use in topical administration. See WO 2006/138468. In such embodiments, the antibiotic can be sulfur antibiotic including without limitation silver sulfadiazine, *i.e*., silvadeen. The co-administered one or more additional agents can be administered concurrently, alternatively or sequentially with the construct.

In other aspects, the disclosure provides a pharmaceutical composition including a therapeutically effective amount of the construct of the invention, and one or more pharmaceutically acceptable diluents, preservatives, solubilizers, emulsifiers, adjuvants, excipients, or carriers. The pharmaceutical composition can be used, for example, for promoting wound healing in a mammal in need thereof. The pharmaceutical composition is particularly useful for promoting diabetic wound healing.

In certain embodiments, the disclosure provides a pharmaceutical composition comprising the compounds of the disclosure together with one or more pharmaceutically acceptable excipients or vehicles, and optionally other therapeutic and/or prophylactic ingredients. Such excipients include liquids such as water, saline, glycerol, polyethylene glycol, hyaluronic acid, ethanol, and the like.

The term "pharmaceutically acceptable vehicle" refers to a diluent, adjuvant, excipient or carrier with which a compound of the disclosure is administered. The terms "effective amount" or "pharmaceutically effective amount" refer to a nontoxic but sufficient amount of the agent to provide the desired biological result. That result can be reduction and/or alleviation of the signs, symptoms, or causes of a disease, or any other desired alteration of a biological system. An appropriate "effective" amount in any individual case can be determined by one of ordinary skill in the art using routine experimentation.

"Pharmaceutically acceptable carriers" for therapeutic use are well known in the pharmaceutical art, and are described, for example, in Remington's Pharmaceutical Sciences, 18th Edition (Easton, Pennsylvania: Mack Publishing Company, 1990). For example, sterile saline and phosphate-buffered saline at physiological pH can be used. Preservatives, stabilizers, dyes and even flavoring agents can be provided in the pharmaceutical composition. For example, sodium benzoate, sorbic acid and esters of p-hydroxybenzoic acid can be added as preservatives. *Id.* at 1449. In addition, antioxidants and suspending agents can be used. *Id.*

Suitable excipients for non-liquid formulations are also known to those of skill in the art. A thorough discussion of pharmaceutically acceptable excipients and salts is available in Remington's Pharmaceutical Sciences, 18th Edition (Easton, Pennsylvania: Mack Publishing Company, 1990).

Additionally, auxiliary substances, such as wetting or emulsifying agents, biological buffering substances, surfactants, and the like, can be present in such vehicles. A biological buffer can be any solution which is pharmacologically acceptable and which provides the formulation with the desired pH, *i.e.,* a pH in the physiologically acceptable range. Examples of buffer solutions include saline, phosphate buffered saline, Tris buffered saline, Hank's buffered saline, and the like.

In certain embodiments, the construct of the invention may be formulated as a topical ointment or cream containing the active ingredient(s), generally in an amount ranging from about 0.01 to about 20% by weight, in some embodiments from about 0.1 to about 20% by weight, in some embodiments from about 0.1 to about 10% by weight, and in some embodiments from about 0.5 to about 15% by weight. When formulated as an ointment, the active ingredients will typically be combined with either a paraffinic or a water-miscible ointment base. Alternatively, the active ingredients may be formulated in a cream with, for example an oil-in-water cream base. Such formulations are well-known in the art and generally include additional ingredients to enhance the dermal penetration or stability of the active ingredients or the formulation.

In various embodiments, the formulations herein can be in the form of aqueous gel, anhydrous gel, a water-in-oil emulsion, oil-in-water emulsion or a suspension. Examples of gel forming procedure for DHEA can be found in US 5,709,878, and US 4,978,532. Gels are semisolid systems of either containing suspended small inorganic particles (two phase gels) or organic macromolecules interpenetrated by a liquid (single phase gels). Emollients such as petrolatum, paraffin wax, beeswax, cetyl palmitate, and lanolin can be included in the formulations herein. When formulated for presentation as a gel, a composition can include a gelling agent such as a finely divided solid and/or a thickener in concentrations that produce a loose molecular network inhibiting the free movement of liquid ingredients. Thus, a typical gel composition includes a concentration of peptide in the range of about 0.1 to about 20 grams per 100 grams of composition, in some embodiments about 0.25 to about 5 grams per 100 grams; a concentration of phospholipid in the range of about 2 to about 50 grams per 100 grams of composition, in some embodiments about 3 to about 25 grams per 100 milliliters; a concentration of finely divided solid in the range of about 0 to about 15 grams per 100 grams of composition, and a concentration of thickener in the range of about 0 to about 15 grams per 100 grams of composition.

Gellants may also be included in the formulations. These agents are typically non-ionic or cationic polymers such as hydroxyethyl cellulose, methylcellulose, guar gum, xanthan gum, hydroxypropylcellulose and cationic cellulosics. A particular example is SEPIGEL^{™}. In one embodiment, a gel comprising a peptide of the invention can be made by mixing a lower alkyl alcohol, a polysorbate, water and the peptide and, optionally, adding and mixing a thickening agent followed by incubating the ingredients until gel formation. Various temperatures may be used for incubation to effect gel formation.

Examples of thickening agents that can be added to the gel or solution formulations described herein include: cellulosic thickening agents, for example, cellulose, hydroxyethyl-cellulose, carboxymethylcellulose, and hydroxypropylmethyl-cellulose; and acrylic thickening agents. Examples of acrylic thickeners are carbomers, for example, non-linear polymers of acrylic acid cross-linked with a polyalkenyl polyether. Examples of carbomers which may be used in the present invention include carboxypolymethylene, carboxyvinyl polymer, and alkyl acrylates, for example, acrylic acid/alkyl methacrylate copolymer. All of the above are available from Noveon, with carboxypolymethylene sold under the tradename CARBOPOL^{®} 980 carboxyvinyl polymer sold as CARBOPOL^{®} 940, and acrylic acid/alkyl methacrylate copolymer sold as PEMULEN^{™} TR-1.

In some embodiments, the formulations of the invention can be applied by misting or spraying the formulation on the skin either via a metered dose device or from a unit dose container. In this method, the formulation can be distributed evenly over a larger area thereby providing a quick means for absorption. Alternatively, the formulation can be applied via an applicator, such as a roll-on applicator, a metered pump dispenser or sponge.

A topical oil-in-water emulsion composition can be prepared by making a solution of comprising a peptide of the invention and adding an immiscible phase (*e.g.*, a biocompatible oil phase) and an optional emulsifying agent. An irritation mitigating agent can also be included, such as C₁₂₋₁₅ alkyl benzoate, octyl methoxycinnamate, octyl dimethyl PABA, octocrylene, menthyl anthranilate, and homomenthyl salicylate.

In certain embodiments a foam comprising compounds of instant application can be prepared. An example of a foam forming procedure can be found in US 7,141,237. For instance, an active agent in a solution as described herein and a quick-breaking foaming agent comprising a mixture of cetyl alcohol and stearyl alcohol, which are dissolved in the ethanol solution can be used. This composition can be packaged in a polyamide-imide-lined aluminum can and pressurized with a propane/butane mixture as the propellant. Under the packaged pressure, the hydrocarbon propellant liquefies and becomes miscible with the water/ethanol solution.

The formulation herein may contain an emulsifier and/or surfactant. A wide variety of such agents can be employed. In one embodiment, the compositions of the present invention comprise from about 0.05% to about 95%, in some embodiments from about 10% to about 80%, and in some embodiments from about 3.5% to about 60% of at least one surfactant. The surfactant, at a minimum, must be hydrophilic enough to disperse in ethanol or other solvent system. The surfactants useful herein can include any of a wide variety of cationic, anionic, zwitterionic, and amphoteric surfactants disclosed in prior patents and other references. The exact surfactant chosen will depend upon the pH of the composition and the other components present.

In one embodiment, the composition comprises a hydrophilic emulsifier or surfactant. In some embodiments, the compositions of the present invention comprise from about 0.05% to about 5%, in some embodiments from about 0.05% to about 3.5% of at least one hydrophilic surfactant. Without intending to be limited by theory, it is believed that the hydrophilic surfactant assists in dispersing hydrophobic materials.

Hydrophilic surfactants are selected from nonionic surfactants. Among the nonionic surfactants that are useful herein are those that can be broadly defined as condensation products of long chain alcohols, e.g., C₈₋₃₀ alcohols, with sugar or starch polymers, *i.e*., glycosides. Representative sugars include glucose, fructose, mannose, and galactose. Examples of long chain alcohols from which the alkyl group can be derived include decyl alcohol, cetyl alcohol, stearyl alcohol, lauryl alcohol, myristyl alcohol, oleyl alcohol, and the like. Commercially available examples of these surfactants include decyl polyglucoside and lauryl polyglucoside.

Other useful nonionic surfactants include the condensation products of alkylene oxides with fatty acids (*i.e*., alkylene oxide esters of fatty acids); the condensation products of alkylene oxides with 2 moles of fatty acids (*i.e*., alkylene oxide diesters of fatty acids); the condensation products of alkylene oxides with fatty alcohols (*i.e.,* alkylene oxide ethers of fatty alcohols); and the condensation products of alkylene oxides with both fatty acids and fatty alcohols. Nonlimiting examples of these alkylene oxide derived nonionic surfactants include ceteth-6, ceteth-10, ceteth-12, ceteareth-6, ceteareth-10, ceteareth-12, steareth-6, steareth-10, steareth-12, PEG-6 stearate, PEG-10 stearate, PEG-100 stearate, PEG-12 stearate, PEG-20 glyceryl stearate, PEG-80 glyceryl tallowate, PEG-10 glyceryl stearate, PEG-30 glyceryl cocoate, PEG-80 glyceryl cocoate, PEG-200 glyceryl tallowate, PEG-8 dilaurate, PEG-10 distearate, and mixtures thereof.

Other nonionic surfactants suitable for use herein include sugar esters and polyesters, alkoxylated sugar esters and polyesters, C₁-C₃₀ fatty acid esters of C₁-C₃₀ fatty alcohols, alkoxylated derivatives of C₁-C₃₀ fatty acid esters of C₁-C₃₀ fatty alcohols, alkoxylated ethers of C₁-C₃₀ fatty alcohols, polyglyceryl esters of C₁-C₃₀ fatty acids, C₁-C₃₀ esters of polyols, C₁-C₃₀ ethers of polyols, alkyl phosphates, polyoxyalkylene fatty ether phosphates, fatty acid amides, acyl lactylates, and mixtures thereof. Nonlimiting examples of these non-silicon-containing emulsifiers include: polyethylene glycol 20 sorbitan monolaurate (Polysorbate 20), polyethylene glycol 5 soya sterol, Steareth-20, Ceteareth-20, PPG-2 methyl glucose ether distearate, Ceteth-10, Polysorbate 80, cetyl phosphate, potassium cetyl phosphate, diethanolamine cetyl phosphate, Polysorbate 60, glyceryl stearate, polyoxyethylene 20 sorbitan trioleate (Polysorbate 85), sorbitan monolaurate, polyoxyethylene 4 lauryl ether sodium stearate, polyglyceryl-4 isostearate, hexyl laurate, PPG-2 methyl glucose ether distearate, PEG-100 stearate, and mixtures thereof. Commercially available surfactants include polysorbate 80 (sold under the tradename TWEEN^{®} 80), polysorbate 20 (sold under the tradename TWEEN^{®} 20), polysorbate 40 (sold under the tradename TWEEN^{®} 40) and polysorbate 60 (sold under the tradename TWEEN^{®} 60). In some embodiments, the surfactants include polysorbates and in some embodiments the surfactant is sold under the tradename TWEEN^{®}.

The construct of this invention can also be administered by a device. Accordingly, administration can be accomplished using a patch either of the reservoir or porous membrane type, or of a solid matrix variety.

A device, or individual dosage unit, can be produced in any manner known to those of skill in the art. After the dermal composition is formed, it may be brought into contact with the backing layer in any manner known to those of skill in the art. Such techniques include calender coating, hot melt coating, solution coating, etc. Backing materials are well known in the art and can comprise plastic films of polyethylene, vinyl acetate resins, ethylene/vinyl acetate copolymers, polyvinyl chloride, polyurethane, and the like, metal foils, non-woven fabric, cloth and commercially available laminates. The backing material generally has a thickness in the range of 2 to 1000 micrometers and the dermal composition is generally disposed on backing material in a thickness ranging from about 12 to 250 micrometers thick.

Suitable release liners are also well known in the art and include the commercially available products of Dow Corning Corporation designated BIO-RELEASE^{™} liner and Syloff7 7610 liner. For embodiments in which a polysiloxane is part of the multiple polymeric adhesive carrier, the release liner must be compatible with the silicone adhesive. An example of a suitable commercially available liner is 3M's 1022 SCOTCH PAK^{™}. The configuration of the transdermal delivery system can be in any shape or size as is necessary or desirable. Illustratively, a single dosage unit may have a surface area in the range of 1 to 200 cm². In some embodiments, sizes are from 5 to 60 cm².

Where the carrier is a flexible, finite polymer, one or more polymers are blended, optionally with PVP to result in a pressure-sensitive adhesive composition, or drug delivery system adhesive system (with incorporated parent drug), which controls delivery of an incorporated parent drug and through the epidermis. A suitable drug delivery system is prepared by mixing a soluble PVP, polyacrylate, polysiloxane, parent drug/prodrug, optional enhancer(s), co-solvent(s), and tackifying agents, if needed, in an appropriate volatile solvent(s), then casting the mixture and removing the solvent(s) by evaporation to form a film. Suitable volatile solvents include, but are not limited to, alcohols such as isopropanol and ethanol; aromatics such as xylenes and toluene; aliphatics such as hexane, cyclohexane, and heptane; and alkanoic acid esters such as ethyl acetate and butyl acetate.

Also included are delivery systems for administration by: passive patches, heated passive patches, passive patches applied onto RF treated skin, and spray-on-skin systems where the total amount applied is fixed and delivery is improved by co-formulated permeation enhancers.

In some embodiments, the composition is administered to the recipient by means of a delivery system or patch. Delivery is accomplished by exposing a source of the substance to be administered to the recipient's skin for an extended period of time. Typically, the formulation is incorporated in or absorbed on a matrix or container from which it is released onto the recipient's skin. The rate of release can be controlled by a membrane placed between the container and the skin, by diffusion directly from the container, or by the skin itself serving as a rate-controlling barrier. Many suitable topical delivery systems and containers therefore are known, ranging in complexity from a simple gauze pad impregnated with the substance to be administered and secured to the skin with an adhesive bandage to multilayer and multi-component structures. Some of the systems are characterized by the use with the substance to be administered of a shaped article sufficiently flexible to snugly fit to the skin of the recipient and thus serve both as container from which the substance is delivered to the recipient's skin and as barrier to prevent loss or leakage of the substance away from the area of the skin to which the substance is to be delivered. A topical delivery system or patch may also contain an added substance that assists the penetration of the active ingredient through the skin, usually termed a skin enhancer or penetration enhancer. The delivery system may contain an ethoxylated oil such as ethoxylated castor oil, ethoxylated jojoba oil, ethoxylated corn oil, and ethoxylated emu oil. An alcohol mixed with the ethoxylated oil may form a penetration enhancer.

Also provided are dosages for the construct. For topical wound healing, one or more doses of about 0.001 mg/cm²-about 10 mg/cm² wound area, about 0.05 mg/cm²-about 5 mg/cm² wound area, about 0.01 mg/cm²-about 1 mg/cm² wound area, about 0.05 mg/cm²-about 0.5 mg/cm² wound area, about 0.01 mg/cm²-about 0.5 mg/cm² wound area, about 0.05 mg/cm²-about 0.2 mg/cm² wound area, or about 0.1 mg/cm²-about 0.5 mg/cm² wound area (or any combination thereof) may be administered to the patient. In certain embodiments, one or more doses of about 0.01 mg/cm², 0.02 mg/cm², 0.03 mg/cm², 0.04 mg/cm², 0.05 mg/cm², 0.06 mg/cm², 0.07 mg/cm², 0.08 mg/cm², 0.09 mg/cm², 0.1 mg/cm², 0.15 mg/cm², 0.2 mg/cm², 0.25 mg/cm², 0.3 mg/cm², 0.4 mg/cm², or 0.5 mg/cm² wound area may be administered to the patient. Such doses may be administered intermittently, e.g., every week or every three weeks (e.g., such that the patient receives from about two to about twenty, or about six doses of the construct). An initial higher loading dose, followed by one or more lower doses may be administered. However, other dosage regimens may be useful. The progress of this therapy is easily monitored by conventional techniques and assays.

The following non-limiting examples are provided to further illustrate the present invention.

### Example 1: Materials and Methods

*Reagents.* PP2 (4-amino-5-(4-chlorophenyl)-7-(t-butyl) pyrazolo [3, 4-d] pyrimidine), the calcium ionophore A23187, *N*^{G}-nitro-L-arginine methyl ester (L-NAME), wortmannin, STO-609, U0126, BSA, and RIPA buffer and F-127 gel sold under the tradename PLURONIC^{®} were from Sigma (St. Louis, MO). n-Octyl-β-D-glucopyranoside (ODG) was from RPI Corp. (Mt Prospect, IL). siRNAs for eNOS, CCR10 and transfection reagent sold under the tradename DHARMAFECT^{®} 1 were from Dharmacon (Lafayette, CO). Transfection reagent sold under the tradename TRANSIT^{®} was from Mirus Bio (Madison, WI). Control siRNA, mouse anti-CCR10 and mouse anti-eNOS antibodies were purchased from Santa Cruz Biotechnologies (Dallas, TX). 4',6-diamidino-2-phenylindole (DAPI), fluorescently labeled secondary antibodies were purchased from Invitrogen (Carlsbad, CA). Mouse anti-eNOS, mouse anti-β-catenin, rabbit and mouse and mouse anti-actin antibodies, human recombinant CCL28 and extracellular matrix sold under the tradename MATRIGEL^{®} were from BD Biosciences (San Diego, CA). Rabbit anti-EEA1, mouse anti-LAMP1 antibodies, mouse anti-Bcl-2, rabbit anti-CD31, rabbit anti-phospho-eNOS (pSer1177), rabbit anti-phosphop85 (pTyr458), rabbit anti-phospho-Src (pTyr416), rabbit anti-phospho-ERK (pT202/Y204) and the corresponding total antibodies, and Griess Reagent kit were from Cell Signaling Technology (Danvers, MA). Skin punch biopsy tool was from Acuderm Inc. (Fort Lauderdale, FL). Hematoxylin, eosin, High-Def and Bluing were from StatLab (McKinney, TX). Transfection reagent sold under the tradename LIPOFECTAMINE^{®} 2000, 4',6-Diamidino-2-phenylindole (DAPI), fluorescently labeled secondary antibodies, DAF-FM Diacetate, nucleic acid isolation reaction sold under the tradename TRIZOL^{®}, and SYBR Green PCR mix were from ThermoFisher Scientific (Waltham, MA). Mouse ELISA kits were purchased from R&D Systems (Minneapolis, MN). Nitrocellulose membrane was from Bio-Rad Laboratories (Hercules, CA). Enhanced chemiluminescent substrate kit sold under the tradename SUPERSIGNAL^{®} West Femto and RESTORE^{™} Western Stripping buffer were from Pierce (Rockford, IL) .

*Cell Culture and Transfection.* Human umbilical vein endothelial cells (HUVECs; Lonza, Walkersville, MD) and human dermal microvascular endothelial cells (HDMVECs; Cell Biologics, Chicago, IL) were cultured in EGM-2 (EGM^{™}-2 plus BULLETKIT^{™}; Lonza, Walkersville, MD) supplemented with 10% (v/v) FBS. cDNA and siRNA transfection in endothelial cells were performed according to known methods (Chen, et al. (2018) Mol. Biol. Cell 29(10):1190-1202; Chen, et al. (2020) FASEB J. 34(4):5838-5850). HEK 293 cells (ATCC, Rockville, MD) and HEK/eNOS cells were cultured in DMEM medium supplemented with 10% FBS and 1% penicillin/streptomycin. cDNA transfection in endothelial cells and HEK cells was respectively carried out with transfection reagents sold under the tradenames TRANSIT^{®} and LIPOFECTAMINE^{®} 2000 according to established methods (Chen, et al. (2018) Mol. Biol. Cell 29(10):1190-1202; Chen, et al. (2020) FASEB J. 34(4):5838-5850; Chen, et al. (2015) Ann. Rheum. Dis. 74:1898-1906). Cells transduced with fluorescently tagged proteins were verified by fluorescence microscopy and immunoblot analysis.

*Construction of CCR10-GFP Plasmid.* To generate the wild-type, C-terminal, GFP-tagged C-C Motif CCR10, full-length *Homo sapiens* CCR10 cDNA (Addgene, Cambridge, MA) was used as a DNA template in a PCR reaction with DNA PHUSION^{®} High-Fidelity Polymerase (New England BioLabs, Ipswich, MA), using the following primer pair lacking the stop codon: CCR10-ATG-EcoRI-F:5'-AAA AAA **GAA TTC** ATG GGG ACG GAG GCC ACA GAG-3' (SEQ ID NO:76) and CCR10-NoStop-KpnI-R:5'-AAA AAA **GGT ACC** AAG TTG TCC CAG GAG AGA CTG TG-3' (SEQ ID NO:77). The resulting PCR fragment was digested with restriction enzymes 5'-EcoRI and 3'-KpnI and ligated using T4 DNA Ligase (New England BioLabs, Ipswich, MA) into pEGFP-N1 vector (Clontech, Mountain View, CA) digested with the same restriction enzymes. The ligated reaction was transformed into Subcloning Efficiency DH5α Competent Cells (ThermoFisher, Waltham, MA).

*Nitric Oxide (NO) Measurement with DAF-FM Diacetate.* Confluent HUVEC monolayers on 96-well plates were loaded with 2.5 µM DAF-FM and incubated for 45 minutes at 37°C. NO concentration was measured using a SpectraMax Microplate Reader (Molecular Devices, San Jose, CA) as described in the art (Chen, et al. (2020) FASEB J. 34(4):5838-5850).

*Nitrite Measurement with Griess Reagent.* Supernatants from confluent endothelial cells on 10-cm dishes with and without stimulation at 37°C were analyzed for nitrite level using a Griess Reagent kit according to the manufacturer's instructions. Nitrite levels were normalized to eNOS expression level in the cells, as described in the art (Chen, et al. (2020) FASEB J. 34(4):5838-5850).

*Immunoblot and Co-Immunoprecipitation (Co-IP).* After serum starvation, inhibitors were added 45 minutes prior to stimulation for indicated times at 37°C, and cells were then collected and lysed for western blot analysis. After probing for p-eNOS (Ser1177), p-p85 (Tyr458), p-Src (Tyr 416), and p-ERK (T202/Y204), the same blots were stripped and re-probed for total proteins mentioned above. For detection of CCL28-induced protein levels of β-catenin and Bcl-2, near-confluent endothelial cells were incubated with 500 ng/ml CCL28 for 24 hours in EGM-2 medium containing 1% FBS.

*Co-IP Experiments.* cells or mouse wounds were collected after treatment, and lysed in 2% ODG in Tris buffer. The homogenates were further prepared for Co-IP experiments as described in the art (Chen, et al. (2018) Mol. Biol. Cell 29(10):1190-1202; Chen, et al. (2020) FASEB J. 34(4):5838-5850; Chen, et al. (2015) Ann. Rheum. Dis. 74:1898-1906).

*Endothelial Cell Tube Formation.* HUVECs were transfected with eNOS (50 nM), CCR10 (100 nM), or scrambled control (100 nM) siRNA and, after 48 hours, the cells were transferred to 96-well plates preloaded with extracellular matrix sold under the tradename MATRIGEL^{®}. To examine the CCL28 signaling pathways, HUVECs were pre-incubated with vehicle or specific inhibitors and then stimulated with or without CCL28 as described previously (Chen, et al. (2015) Ann. Rheum. Dis. 74:1898-1906).

*Confocal Microscopy.* For cellular immunostaining, confluent monolayers of cells grown on coverslips were prepared as described previously (Chen, et al. (2012) Mol. Biol. Cell 23(7):1388-1398). Fluorescent images were obtained using a Zeiss LSM 880 confocal microscope. The co-localization coefficient in specified regions of interest (ROI) were determined using Zeiss Zen software. Briefly, using the Overlay tool, the desired ROI was drawn along the cell edge and the values for the ROIs tabulated. The scatterplot and table were automatically adjusted to the pixel distribution for the selected ROI. The data from different treatment groups were collected and analyzed for statistical significance as described previously (Chen, et al. (2020) FASEB J. 34(4):5838-5850).

*Synthesis of Myristoylated Peptides.* Myristoylated peptides were synthesized using a step-wise solid-phase method using 9-fluorenylmethoxycarbonyl (Fmoc) chemistry on a Wang resin (AnaSpec, Fremont, CA) with a 12-channel multiplex peptide synthesizer (Protein Technologies, Tucson, AZ) as described previously (Chen, et al. (2020) FASEB J. 34(4):5838-5850; Jayathilaka, et al. (2007) J. Biomol. Tech. 18:46).

*Mouse Healing Models.* All animal procedures were performed in accordance with the Guide for the Care and Use of Laboratory Animals (National Institutes of Health). *WT C57BL*/*6, eMOS*^{*-*/*-*} and *db*/*db* mice were purchased from Jackson Laboratory (Bar Harbor, ME). *CCR10*^{*-*/*-*} mice have been described in the art, see, e.g., Jin, et al. (2011) J. Immunol. 185(10):5723-31. Four 5-mm or two 8-mm full thickness excisional wounds were made on the dorsal skin of mice (male, aged at 9 weeks) using a standard skin biopsy punch under ketamine (100 mg/kg) and xylazine (5 mg/kg) anesthesia. Scrambled myristoylated (Myr-) control or Myr-CBD7 peptides were applied topically to wounds in F-127 gel sold under the tradename PLURONIC^{®} after wounding (Mirza, et al. (2009) Am. J. Pathol. 175:2454-2462). Wound size was determined as previously described (Chen, et al. (2020) FASEB J. 34(4):5838-5850; Zhao, et al. (2016) PLoS ONE. 11:e0146451) and wounds were collected at indicated times for indicated measurements.

*Hematoxylin and Eosin (H&E) Staining.* H&E staining was performed as described previously (Chignalia, et al. (2015) Am. J. Pathol. 185:1251-1263). Slides with 5 µm tissue sections were baked at 60°C for 30 minutes and stained with an Autostainer XL (Leica Microsystems, Wetzlar, Germany) using preset protocol. Briefly, slides were deparaffinized in three changes of xylene, rehydrated in 100% and 95% ethanol, incubated for 1.5 minutes followed by a brief immersion in High-Def and Bluing to obtain crisp nuclear details. Slides were then incubated in eosin for 2 minutes, dehydrated and mounted with Micromount media (Leica Biosystems). Whole slide images were obtained using Aperio AT2 brightfield scanner (Leica Microsystems, Wetzlar, Germany).

*CD31 Staining in Mouse Wound Tissue.* Formalin-fixed mouse skin samples were processed on ASP300 S automated tissue processor (Leica Biosystems, Buffalo Grove, IL) using a standard overnight processing protocol and embedded into paraffin blocks. Tissue was sectioned at 5 µm and stained with rabbit anti-CD31 antibody. Sections were deparaffinized, rehydrated, and stained on BOND^{™} RX automated stainer (Leica Biosystems, Buffalo Grove, IL) using a preset protocol. In brief, sections were subjected to EDTA-based antigen retrieval with BOND^{™} ER2 solution (Leica Biosystems, Buffalo Grove, IL) for 20 minutes at 100°C. Endogenous peroxidase activity and non-specific binding was blocked by treating samples with peroxidase block and protein block with Background Sniper (Biocare Medical, Pacheco, CA) for 15 minutes and 30 minutes at room temperature, respectively. Sections were then incubated with the primary antibody diluted at 1:200 for 30 minutes at room temperature. BOND^{™} Polymer Refine Detection kit (Leica Biosystems, Buffalo Grove, IL) was used for detection. All sections were then counterstained with hematoxylin for 10 minutes and mounted with Micromount media (Leica Biosystems, Buffalo Grove, IL). Whole slide images were acquired at 20X magnification on Aperio AT2 digital automated scanner (Leica Biosystems, Buffalo Grove, IL).

*Real-Time RT-PCR.* Total cellular RNA from mouse skin wounds was extracted using nucleic acid extraction reagent sold under the tradename TRIZOL^{®}. mRNA expression of different genes was examined by real-time RT-PCR that employed a SYBR Green PCR mix. Relative gene expression was determined by the ΔΔCₜ method based on GAPDH levels as described previously (Chen, et al. (2013) J. Immunol. 190:5256-5266).

*ELISA Measurement.* Mouse skin wounds were collected, lysed, and prepared for ELISA measurement of mouse CCL28 and IL-6 according to manufacturer's instructions.

*Blood Perfusion Measurement.* Immediately after creation of dorsal skin wounds on *db*/*db* mice using an 8 mm punch, 50 µl of 50 µM Myr-scrambled control or Myr-CBD7 peptide in F-127 gel sold under the tradename PLURONIC^{®} was applied topically to the wounds on *db*/*db* mice. Blood perfusion was analyzed based on motion detected below the surface of the wound using a near infrared laser based PeriCam PSI laser speckle contrast analysis system (LASCA) as described previously (Elgharably, et al. (2013) Wound Repair Regen. 21(3):473-481; Das, et al. (2016) J. Immunol. 196 (12):5089-5100).

*Statistical Analysis.* Data are expressed as mean ± SEM from at least three independent experiments. Statistical analysis was performed by Student's t-test or one-way ANOVA using GraphPad InStat software (San Diego, CA). Statistical significance was defined as *P* < 0.05.

### Example 2: CCL28 activated eNOS-dependent Src, PI3K and MAPK signaling pathways in human endothelial cells

CCR10 levels were found to be highly expressed in human umbilical vein endothelial cells (HUVECs) and endothelial progenitor cells (EPCs). NO production was also observed in HUVECs following treatment with 500 ng/ml human recombinant CCL28, the ligand for CCR10. The signaling pathways activated by CCL28 binding to CCR10 in endothelial cells were further investigated. Phosphorylation of eNOS (Ser1177), p85 (Tyr458; PI3K), Src kinase (Tyr418), and ERK (T202/Y204; MAPK) were all significantly increased following CCL28 treatment for up to 60 minutes. However, when eNOS was depleted with siRNA, phosphorylation of p85, Src, and MAPK were abolished indicating that CCL28 promotes eNOS-dependent Src, PI3K and MAPK signaling pathways in endothelial cells.

The role of CCL28/CCR10 signaling pathways was further investigated in angiogenesis. Pretreatment of cells with 10 µM wortmannin (PI3K inhibitor), 5 µM U0126 (MAPK/ERK inhibitor), 1 mM L-NAME (eNOS inhibitor), 10 µM PP2 (Src kinase inhibitor), and 10 µM STO-609 (CaMKII inhibitor) reduced endothelial cell tube formation induced by 500 ng/ml CCL28. These results indicate that eNOS/NO, Src, PI3K and MAPK mediate, at least in part, CCL28-dependent angiogenesis. In addition, CCL28 stimulation of endothelial cells for 24 hours enhanced expression levels of cell adhesion protein β-catenin and anti-apoptotic protein B-cell leukemia/lymphoma-2 (Bcl-2), supporting the proliferation potential of endothelial cells in response to stimulation with CCL28.

### Example 3: CCR10 regulation of eNOS-dependent angiogenesis in HUVECs

To further investigate the role of CCR10 and eNOS in endothelial cell tube formation, siRNA targeting CCR10 (100 nM) or eNOS (50 nM) were used and tube formation measured in HUVEC. This analysis indicated that knockdown of either CCR10 or eNOS abolished endothelial cell tube formation, indicating both CCR10 and eNOS proteins play a significant role in angiogenesis. It was shown by western blot analysis that eNOS knockdown did not affect CCR10 expression, however, CCR10 knockdown reduced eNOS expression level by 60%. The regulation of eNOS by CCR10 in endothelial cells was further demonstrated by confocal microscopy in that eNOS co-localized with CCR10 in control siRNA-treated cells. Consistent with western blot results, CCR10 fluorescence intensity was not affected in eNOS-depleted cells, however, the fluorescence intensity of eNOS dramatically decreased in CCR10 depleted cells. These data may indicate that eNOS depletion in endothelial cells treated with CCR10 siRNA reflects the stabilization of the pool of eNOS that co-localizes with CCR10. Taken together, these results indicate that CCR10, via regulation of eNOS expression and activation, plays an important role in angiogenesis.

### Example 4: Regulation of eNOS expression and function by CCR10

Since eNOS expression can be regulated by CCR10 in primary culture human endothelial cells, the effect of CCR10 expression on eNOS activity in human endothelial cells was further investigated. After treatment with 100 nM CCR10 siRNA for 72 hours, endothelial cells were stimulated with 5 µM calcium ionophore A23187 and cell lysates were collected and prepared for western blot analysis. Both CCR10 and eNOS expression levels were reduced. However, phosphorylation of eNOS was significantly increased compared to control siRNA, even at 0 minutes, indicating that CCR10 binding basally suppresses eNOS activity. Nitrite concentration in cell supernatants was also measured and eNOS activity was calculated relative to total eNOS expression. Consistent with increased eNOS phosphorylation, NO production was significantly higher per molecule of eNOS in endothelial cells after CCR10 knockdown indicating that CCR10 negatively regulates eNOS expression and activity in endothelial cells.

### Example 5: Ligand-induced interaction between CCR10 and eNOS

To investigate whether CCR10 interacts directly with eNOS, primary culture HUVEC were collected and prepared for co-immunoprecipitation (Co-IP) to assess CCR10-eNOS interaction following stimulation with 5 µM A23187. Enhanced binding of CCR10 to eNOS was observed in endothelial cells stimulated with A23187 based on Co-IP with anti-CCR10 antibody or anti-eNOS antibody, where maximum binding was observed 5 minutes after addition of A23187. Similar results were obtained in endothelial cells following stimulation with 500 ng/ml human recombinant CCL28. CCR10-eNOS interaction was further supported by confocal microscopy and high-resolution imaging. Upon stimulation with A23187 or CCL28 for 5 minutes, CCR10 co-localized to a greater extent with eNOS on the plasma membrane. Co-localization coefficient in the regions of interest (ROI) in the plasma membrane were quantified from Zeiss LSM 880 confocal microscope images using Zeiss Zen software. Co-localization coefficient of CCR10 and eNOS was significantly greater following A23187 (81%) and CCL28 (78%) stimulation as compared to untreated control cells (43%). These results indicate increased interaction between CCR10 and eNOS at or near the plasma membrane of activated endothelial cells.

### Example 6: Blockade of eNOS-CCR10 interaction with myristoylated CCR10 binding domain (Myr-CBD) peptides enhances eNOS activity

Results indicated that CCR10 may directly interact with eNOS in endothelial cells, that CCR10 regulates eNOS expression and angiogenesis and inhibits eNOS activity. Therefore, the effect of inhibition of CCR10-eNOS interaction was investigated using a panel of cell-permeable peptides based on the sequence of the predicted interaction domain on eNOS. Similar peptides have been described in determining the solution structure of calmodulin bound to the target peptide of eNOS (Piazza, et al. (2014) Biochemistry 53(8):1241-9; Piazza, et al. (2016) Biochemistry 55(42):5962-71). First, myristoylated 20 amino acid peptide (491-TRKKTFKEVANAVKISASLM-510 (P1); SEQ ID NO:61) was synthesized based on the sequence of human eNOS (which is conserved in mouse eNOS). Co-IP experiments in HUVECs indicated that pretreatment with 50 µM cell permeable 20 amino acid myristoylated CCR10 binding domain peptide (Myr-CBD20) significantly reduced CCR10-eNOS interaction in endothelial cells stimulated with 5 µM A23187 for 5 minutes compared with control peptide. It was subsequently determined whether truncated versions of the CBD-derived peptide would have the same effect. Accordingly, the truncated peptides listed in Table 3 were synthesized. P1, P2 and P3 are comparative examples. P4 is the construct of the invention.

**TABLE 3**

| Peptide | Designation | Residues* | Sequence | SEQ ID NO: |
|---|---|---|---|---|
| P1 | Myr-CBD20 | 491-510 | TRKKTFKEVANAVKISASLM | 61 |
| P2 | Myr-CBD15 | 496-510 | FKEVANAVKISASLM | 62 |
| P3 | Myr-CBD12 | 499-510 | VANAVKISASLM | 63 |
| P4 | Myr-CBD7 | 504-510 | KISASLM | 64 |

| | | | | |
|---|---|---|---|---|
| *Relative to full-length human eNOS (GENBANK Accession No. NP_000594). | | | | |

Pretreatment with 50 µM P1, P2, P3 or P4 completely blocked CCR10-eNOS interaction in endothelial cells following stimulation with 5 µM A23187 for 5 minutes. Given that the Myr-CBD7 peptide exhibited significant inhibition, the effect of Myr-CBD7 peptide on eNOS activity was assessed. Co-IP experiments showed significant reduction of CCR10-eNOS interaction in cells treated with Myr-CBD7 compared with scrambled control peptide (Myr-MSIALKS; SEQ ID NO:5). Consistent with reduction in negative regulation by CCR10-dependent binding, eNOS phosphorylation (Ser1177) was significantly enhanced in presence of Myr-CBD7.

It has been reported that calmodulin (CaM) binding to bovine eNOS occurs at residues 493-TRKKTFKEVANAVKISASLM-512 (SEQ ID NO:2) (Venema, et al. (1996) J. Biol. Chem. 271(11):6435-6440; Venema, et al. (1995) J. Biol. Chem. 270:14705-14711). Therefore, using Co-IP, it was assessed whether Myr-CBD7 affects eNOS-CaM binding. This analysis indicated that pretreatment of endothelial cells with Myr-CBD7 peptide had no effect on CaM-eNOS binding induced by a 5-minute treatment with 500 ng/ml CCL28. Thus, these data indicate that the 504-KISASLM-510 (SEQ ID NO:1) domain in human eNOS is a specific binding region for CCR10.

### Example 7: Topical administration of Myr-CBD7 peptide improved dermal wound healing in mice

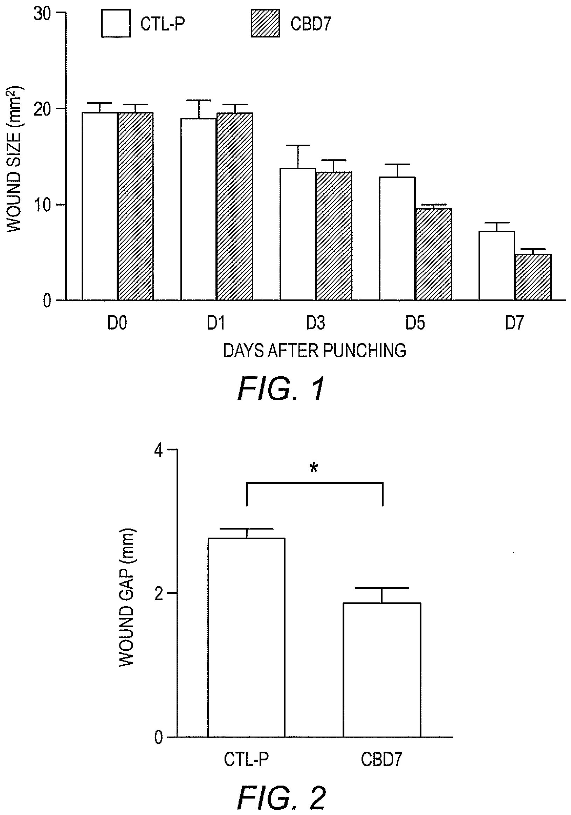

CCL28 (also called mucosa-associated epithelial chemokine) is a CC chemokine that signals via CCR10 as well as CCR3 (Pan, et al. (2000) J. Immunol. 165:2943). CCR10 has been reported to have two functional ligands, CCL27 and CCL28 that are involved in the epithelial immunity (Xiong, et al. (2012) Protein Cell 3:571-580). It was observed that CCL28 and CCR10 levels were highly expressed compared to CCL27 and CCR3 in skin of WT C57BL/6 mice. Similar to IL-6, the protein level of CCL28 determined by ELISA was elevated significantly at day 3 and day 7 in mouse wounds. Therefore, it was subsequently investigated whether blockade of CCR10-eNOS interaction with Myr-CBD7 peptide has an effect on wound healing in WT C57BL/6 mice. Four 5 mm diameter full thickness excisional wounds were made on the mouse dorsal skin and 30 µl Myr-CBD7 peptide (50 µM) was topically administrated onto the wounds immediately after creating the punch wound. Compared to control peptide treated wounds, Myr-CBD7 peptide significantly reduced wound size by 20% on day 5 and by 35% on day 7 (FIG. 1). Wound size reduction with Myr-CBD7 treatment was associated with re-epithelization of the skin wound observed in H&E stained wound sections (FIG. 2).

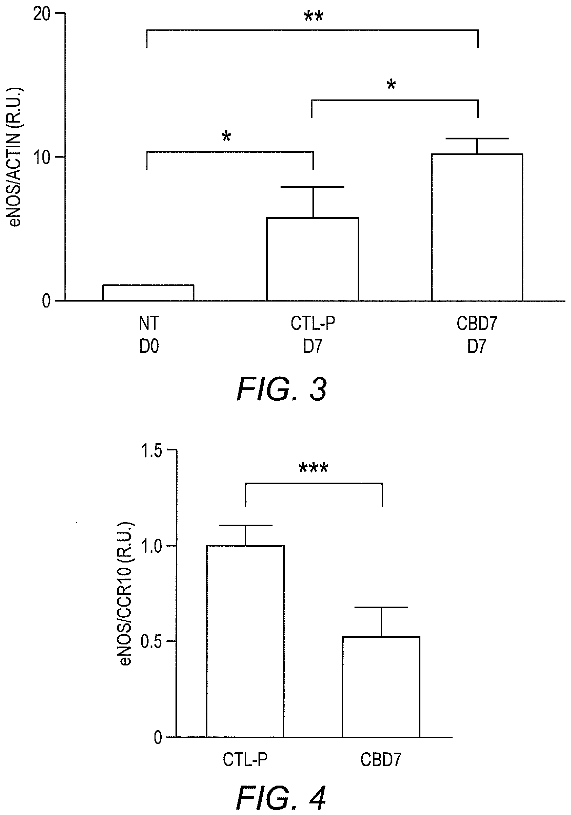

Myr-CBD7 peptide treatment also enhanced eNOS protein level in wounds on day 7 (FIG. 3) compared to control peptide, and Co-IP showed that Myr-CBD7 peptide treatment reduced CCR10-eNOS interaction in the wounds on day 7 (FIG. 4). Besides eNOS, mRNA level of another endothelial cell marker, CD31, was also elevated; CCR10 mRNA level, however, did not change. In addition, Myr-CBD7 peptide treatment resulted in reduction of mRNA levels of CCL28 and proinflammatory cytokine IL-6 while enhancing anti-inflammatory cytokine IL-4 mRNA in the mouse dermal wounds on day 7. The mRNA level of hepatocyte growth factor (HGF) increased, albeit non-significantly, by 35% in Myr-CBD7 treated wounds. Taken together, these results indicate that topical treatment of mouse skin wounds with Myr-CBD7 peptide enhances eNOS/NO levels by blocking CCR10-eNOS interaction thereby facilitating an anti-inflammatory environment that promotes wound angiogenesis and wound healing.

### Example 8: Reduced eNOS expression and elevated level of CCR10 in type 2 diabetes mellitus (T2DM) patients and genetically diabetic mice

It has been shown that plasma nitric oxide (NO) concentration is higher in lean healthy controls (LHCs) than in T2DM patients in the basal state (37.4 ± 10.1*µ*mol/L vs. 21.6 ± 3.9 pmol/L; Mahmoud, et al. (2016) Physiol. Rep. 4:pii:e12895). In the present analysis, the expression levels of eNOS, an important endothelial marker protein, was further assessed in human skeletal muscle biopsies. Protein levels of eNOS were significantly lower in T2DM patients compared to LHC. Subsequently, the level of chemokine receptor CCR10 in these human biopsies was measured. mRNA levels of CCR10 were also determined with real-time RT-PCR and it was observed to be significantly elevated in biopsies from T2DM relative to LHC. The expression levels of eNOS and CCR10 were also measured in diabetic *db*/*db* mice, a genetically leptin receptor db mutant (LEPR^{db}) mouse used as a spontaneous model of type 2 diabetes. Heavier body weight, elevated blood glucose, and increased expression of pro-inflammatory cytokines in dorsal skin were observed in *db*/*db* mice compared to *WT C57BL*/*6* mice. In dorsal skin of *db*/*db* mice, eNOS expression was significantly reduced compared to *WT* mice and CCR10 mRNA was significantly elevated compared to *WT.* Taken together, reduced eNOS and elevated CCR10 levels observed in human T2DM patients and genetically obese and diabetic *db*/*db* mice indicate that CCR10 may play an important role in regulating eNOS expression and associated with the pathological etiology of cardiovascular dysfunction in type 2 diabetics.

### Example 9: Downregulation of eNOS by CCR10 overexpression in mouse dorsal skin and human endothelial cells

As determined by real-time RT-PCR, expression of CCR3, another receptor for CCL28, was much lower in dorsal skin of both *WT* and *db*/*db* mice compared to CCR10. Thus, CCR10 was considered to be the primary CCL28 receptor in further studies. Dorsal skin from four different mouse strains, *eNOS*^{*-*/}*⁻, db*/*db, WT,* and *CCR10*^{*-*/}*⁻,* were collected and prepared for determination of eNOS and CCR10 expression. Western blot analysis showed reduced eNOS expression in *db*/*db* mice. Interestingly, eNOS expression levels were elevated in *CCR10*^{*-*/*-*} mice. Furthermore, CCR10 mRNA levels in mouse skin determined by real-time RT-PCR was surprisingly elevated in *eMOS*^{*-*/*-*} mice. Thus, when eNOS expression is reduced, as in *eMOS*^{*-*/*-*} and db/db, CCR10 level is increased, and when CCR10 is reduced, as in *CCR10*^{*-*/*-*} mice, eNOS protein levels are increased. These data indicate there is a reciprocal relationship between eNOS and CCR10 expression.

Wound healing time in db/db and WT mice was also compared to *eNOS⁻ⁱ⁻* and *CCR10*^{*-*/*-*} mice. Wounds were produced on the dorsal skin and images were collected every 48 hours and analyzed. Delayed wound healing was observed in *eNOS*^{*-*/}*⁻, db*/*db* and *CCR10*^{*-*/*-*} mice starting from day 3, compared to *WT.* Importantly, delayed healing in diabetic *db*/*db* mice (85%; relative to day 0) was very similar to that observed in *eMOS*^{*-*/*-*} mice (70%) on day 7, while *CCR10*^{*-*/*-*} mice (26%) showed a relatively larger wounds than *WT* mice (16%).

It has been shown that CCR10 is highly expressed in human umbilical vein endothelial cells (HUVECs) and human endothelial progenitor cells (EPCs) (Chen, et al. (2020) FASEB J. 34(4):5838-5850). In order to understand whether the CCL28/CCR10 signaling axis in dermal microvessels plays a role in wound healing, 500 ng/ml CCL28 was administrated to human dermal microvascular endothelial cells (HDMVECs). eNOS-CCR10 interaction increased with maximum binding observed at 5 minutes, similar to that observed in HUVEC (Chen, et al. (2020) FASEB J. 34(4):5838-5850). CCR10-eNOS interaction was further demonstrated using confocal microscopy. Upon stimulation with CCL28 for 5 minutes, CCR10 co-localized to a greater extent with eNOS on the plasma membrane.

In order to understand whether eNOS is regulated by CCR10 in dermal endothelial cells, CCR10-GFP cDNA was transfected into primarily culture HDMVECs. With increasing amounts of CCR10-GFP cDNA transfected, eNOS expression levels significantly decreased, and consistent with a reduction in eNOS expression, overexpression of CCR10 also reduced tube formation of HDMVECs. Reduced eNOS expression and NO production were also observed in stably-transfected HEK/eNOS cells further transduced with CCR10-GFP cDNA. However, overexpression of eNOS had no effect on CCR10 levels in stable HEK/CCR10-GFP cells. Taken together, these results indicate that overexpression of CCR10 leads to downregulation of eNOS and that this is associated with reduced angiogenesis *in vitro.*

### Example 10: Internalization of eNOS by CCR10 can be prevented by Myr-CBD7 peptide in HDMVECs stimulated with CCL28

With persistent stimulation, GPCRs are phosphorylated by G protein receptor kinase's (GRKs) that lead to the recruitment of β-arrestin which mediates receptor desensitization/downregulation. GPCRs internalized via clathrin- or caveolae-mediated endocytosis and targeted to lysosomes for degradation or dephosphorylated and recycled back to the cell surface to enable a new round of activation. In preliminary analyses, eNOS showed more co-localization with EEA1 (early endosome antigen 1) in HDMVECs following treatment with 500 ng/ml CCL28 for 90 minutes, indicating that CCL28-activated CCR10 in endothelial cells leads to eNOS internalization. Following a longer treatment time (3 hours) with CCL28, co-IP experiments indicated that both eNOS and CCR10 were associated with LAMP1 (lysosomal-associated membrane protein 1). These results indicated that both proteins undergo degradation as compared to total cellular protein levels which did not change.

Since myristoylated 7 amino acid CCR10-binding domain (Myr-CBD7) peptide can block eNOS interaction with CCR10 and increase eNOS activity in HUVECs, it was assessed whether Myr-CBD7 can prevent eNOS internalization and degradation in HDMVECs following CCL28 stimulation. Pretreatment with Myr-CBD7 prevented eNOS, but not CCR10, interaction with LAMP1 compared to scrambled control peptide, while total protein levels did not change. Confocal microscopy further confirmed that eNOS localization remained on the plasma membrane of endothelial cells following pretreatment with Myr-CBD7 prior to stimulation with CCL28 for 3 hours, while eNOS co-localized with LAMP1 in perinuclear regions in cells pretreated with control peptide. In addition, in HEK/eNOS cells with/without co-expression of CCR10-GFP, NO production was elevated significantly by pretreatment with Myr-CBD7 compared with control peptide. These data indicate that eNOS, due to its interaction with CCR10, undergoes internalization and degradation following activation of endothelial cells with CCL28. However, blockade of eNOS-CCR10 interaction with Myr-CBD7 peptide leads to maintenance of eNOS on cell surface and the ability to activate eNOS and increase NO production.

### Example 11: Topical application of Myr-CBD7 on dorsal skin wounds of diabetic db/db mice improved wound healing by enhancing eNOS/NO level and microvessel density

Topical administration of Myr-CBD7 peptide on dermal wounds of *WT C57BL*/*6* mice decreased wound healing time. Furthermore, treatment with Myr-CBD7 not only enhanced expression of eNOS, CD31, and IL-4, it also reduced CCL28 and IL-6 levels in the wounds. Accordingly, the effect of Myr-CBD7 peptide on restoration of eNOS/NO levels and dorsal skin wound healing of *db*/*db* mice, which exhibited obesity and hyperglycemia, was further investigated.

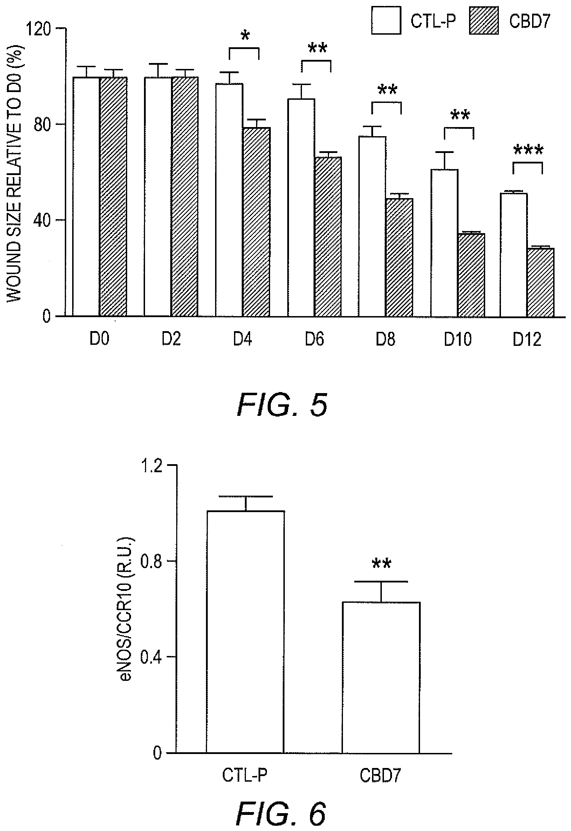

Two 8 mm full thickness excisional wounds were made on the dorsal skin of diabetic *db*/*db* mice and then 50 µl of 50 µM Myr-CBD7 peptide was topically applied. As shown in FIG. 5, Myr-CBD7 peptide treatment significantly decreased wound healing time by 4 days (day 8, 10 and 12 wounds treated with control peptide were equivalent to day 4, 6 and 8 wounds treated with Myr-CBD7, respectively). Thus, Myr-CBD7 reduced wound size significantly starting on day 4 (by 19%) which continued through day 12 (by 46%), compared to control peptide (FIG. 5). eNOS expression and NO production were significantly elevated in wounds on day 12 following treatment with Myr-CBD7 peptide as determined by western blot analysis and nitrite measurement, respectively. Reduced CCR10-eNOS interaction was also observed in the mouse wounds following treatment with Myr-CBD7 peptide as determined by Co-IP (FIG. 6). Further, immunohistochemistry showed enhanced microvessel density (CD31 staining) in mouse wounds on day 12 following Myr-CBD7 treatment. These data indicated that Myr-CBD7 peptide treatment prevents eNOS inhibition and internalization by CCR10 thereby facilitating NO production and angiogenesis associated with improved wound healing in diabetic *db*/*db* mice.

### Example 12: Alteration of wound microenvironment from proinflammatory (M1) to anti-inflammatory (M2) in db/db mice following topical administration of Myr-CBD7 peptide

ELISA measurement showed that higher levels of proinflammatory cytokines IL-6, TNF-α and IL-1β as well as VEGF in the dorsal skin of *db*/*db* mice, compared to *WT* mice. It was investigated whether topical application of Myr-CBD7 affects these factors in *db*/*db* mice known to play important roles in the healing processes. Mouse wounds on day 3 (Inflammation Phase) and day 12 (Proliferation Phase) were collected and inflammatory factor levels were determined by ELISA and real-time RT-PCR.

ELISA measurement demonstrated significantly reduced protein levels of CCL28, and pro-inflammatory cytokines IL-6, TNF-α, IL-1β in day 3 *db*/*db* mouse wounds following treatment with Myr-CBD7. Consistent with protein levels, mRNA levels of IL-6 were also reduced. In addition, the level of CCR10, which was higher in *db*/*db* dorsal skin compared to WT, was also reduced on day 3 following treatment with Myr-CBD, as compared to control peptide.

On day 12 after Myr-CBD7 treatment, elevated levels of eNOS/NO were observed as well as enhanced angiogenesis in mouse wounds. Furthermore, Myr-CBD7 treatment lead to elevated levels of VEGF, anti-inflammatory cytokines IL-13 and IL-4 (M2 markers), as well as EC markers eNOS, CD31, Flk-1 and VE-cadherin in mouse wounds.

To further investigate if Myr-CBD7 promotes healing of dorsal skin wounds on *db*/*db* mice by stimulating angiogenesis, blood perfusion as detected by laser speckle contrast analysis (LASCA), was monitored. As above, 50 µM Myr-CBD7 or control peptide was administered to wounds and on day 7, when wound size was significantly reduced, blood perfusion in the same wounds was observed to be significantly increased. These results indicate that inhibition of CCR10 binding to eNOS in *db*/*db* mouse skin wounds stimulates wound perfusion and promotes healing.

Taken together, these data indicate that blocking CCR10-eNOS interaction with Myr-CBD7 peptide upregulated eNOS/NO bioavailability which leads to a switch in the wound microenvironment from pro-inflammatory (M1) to anti-inflammatory (M2) that facilitates angiogenesis, blood perfusion, and improved wound healing.

## Claims

1. A construct consisting of myr-KISASLM (SEQ ID NO:64).

2. A pharmaceutical composition comprising the construct of claim 1 and a pharmaceutically acceptable vehicle.

3. The pharmaceutical composition of claim 2, wherein said pharmaceutical composition is formulated for topical administration.

4. A pharmaceutical composition for use in a method of promoting or accelerating wound healing, the use comprising administering to a subject in need of treatment an effective amount of the pharmaceutical composition of claim 2 thereby promoting or accelerating wound healing in the subject.

5. The pharmaceutical composition for use of claim 4, wherein the subject is diabetic.

6. The pharmaceutical composition for use of claim 4, wherein the wound is a diabetic foot ulcer.

7. The pharmaceutical composition for use of claim 4, wherein the subject is co-administered at least one additional therapeutic agent for improving or accelerating wound healing.

8. The pharmaceutical composition for use of claim 4, wherein the pharmaceutical composition is administered topically.

## Patentansprüche

1. Konstrukt, bestehend aus myr-KISASLM (SEQ ID NO:64).

2. Pharmazeutische Zusammensetzung, umfassend das Konstrukt nach Anspruch 1 und einen pharmazeutisch akzeptablen Träger.

3. Pharmazeutische Zusammensetzung nach Anspruch 2, wobei die pharmazeutische Zusammensetzung für topische Verabreichung formuliert ist.

4. Pharmazeutische Zusammensetzung zur Verwendung in einem Verfahren zum Fördern oder Beschleunigen von Wundheilung, wobei die Verwendung Verabreichen einer wirksamen Menge der pharmazeutischen Zusammensetzung nach Anspruch 2 an ein behandlungsbedürftiges Subjekt umfasst, wodurch Wundheilung in dem Subjekt gefördert oder beschleunigt wird.

5. Pharmazeutische Zusammensetzung zur Verwendung nach Anspruch 4, wobei das Subjekt diabetisch ist.

6. Pharmazeutische Zusammensetzung zur Verwendung nach Anspruch 4, wobei die Wunde ein diabetisches Fußulkus ist.

7. Pharmazeutische Zusammensetzung zur Verwendung nach Anspruch 4, wobei dem Subjekt mindestens ein zusätzliches therapeutisches Mittel zum Verbessern oder Beschleunigen von Wundheilung mitverabreicht wird.

8. Pharmazeutische Zusammensetzung zur Verwendung nach Anspruch 4, wobei die pharmazeutische Zusammensetzung topisch verabreicht wird.

## Revendications

1. Construction composée de myr-KISASLM (SEQ ID NO:64).

2. Composition pharmaceutique comprenant la construction selon la revendication 1 et un véhicule pharmaceutiquement acceptable.

3. Composition pharmaceutique selon la revendication 2, dans laquelle ladite composition pharmaceutique est formulée pour une administration topique.

4. Composition pharmaceutique pour une utilisation dans un procédé favorisant ou accélérant la cicatrisation d'une plaie, l'utilisation comprenant l'administration, à un sujet nécessitant un traitement, d'une quantité efficace de la composition pharmaceutique selon la revendication 2, favorisant ou accélérant ainsi la cicatrisation de la plaie chez le sujet.

5. Composition pharmaceutique pour une utilisation selon la revendication 4, dans laquelle le sujet est diabétique.

6. Composition pharmaceutique pour une utilisation selon la revendication 4, dans laquelle la plaie est un ulcère du pied diabétique.

7. Composition pharmaceutique pour une utilisation selon la revendication 4, dans laquelle au moins un agent thérapeutique supplémentaire est co-administré au sujet pour améliorer ou accélérer la cicatrisation de la plaie.

8. Composition pharmaceutique pour une utilisation selon la revendication 4, dans laquelle la composition pharmaceutique est administrée par voie topique.
